# EUROPEAN PATENT APPLICATION

(11) **EP 1 112 729 A1**
(43) Date of publication of application: **04.07.2001**
(21) Application number: 99125937.5
(22) Date of filing: 23.12.1999
(51) Int. Cl.: A61F 13/15, A61F 5/455

(54) **Liquid removal system having reduced dimensions and reduced weight**

(71) Applicant: THE PROCTER & GAMBLE COMPANY, Cincinnati, Ohio 45202 (US)
(72) Inventor: Schmidt, Matthias, 65510 Idstein (DE); Ehrnsperger, Bruno Johannes, 65936 Frankfurt (DE)
(74) Representative: Kohol, Sonia

(57) **Abstract**

The present invention provides a liquid removal system for applications where there is a need for liquid removal including laboratories and workshops as well as medical applications and dental applications. The interface device of the liquid removal system of the present invention weighs less than 500 grams and therefore provides increased wearing comfort to the wearer.

## Description

### Field of the invention

The present invention provides a liquid removal system for applications where there is a need for liquid removal including laboratories and workshops as well as medical applications and dental applications.

### Background

Articles to manage body exudated such as urine are well known in the art. In this context, managing body exudated includes acquiring, distributing, and storing body exudated such as urine, menses, fecal material, and the like. A wide variety of such articles has been proposed including diapers, sanitary napkins, adult incontinence articles such as briefs or bed mats, underarm sweat pads, catheters, bottles, bed pans, and the like. Of course, similar articles have also been used for the acquisition of other fluids such as for spill removal in laboratories and workshops.

In United States patents No. 5,678,564 (Lawrence et al.) and No. 5,911,222 (Lawrence et al.) a liquid removal system having an interface device and a vacuum source is described. The interface device has a porous membrane with an entrance zone on one side. The vacuum source maintains a vacuum on the side of the membrane opposite the entrance zone when the membrane is wetted. Liquid which contacts the wetted porous membrane is removed from the interface device by the vacuum source. This liquid removal system and in particular its interface device is, however, impractical for most applications due to its large dimensions and its heavy weight.

It is therefore an object of the present invention to provide a liquid removal system which overcomes the problems posed by the prior art liquid removal systems.

It is a further object of the present invention to provide a liquid removal system which has small dimensions and a light weight.

### Summary of the invention

The present invention provides a liquid removal system which comprises an interface device, said interface device comprising
-- a first zone and
-- a second zone and having
-- a porous membrane separating said first zone from said second zone,
said second zone being adapted to be connected to a suction source, said porous membrane being capable of maintaining a suction in said second zone without permitting air from said first zone to pass through said membrane into said second zone when said membrane has been wetted with a first liquid; said suction being maintained until said membrane is contacted with a second liquid; and wherein said second liquid upon entering said first zone and contacting said porous membrane is removed from said first zone by said suction in said second zone by passing through said membrane into said second zone, said first zone having a user facing surface, said second zone having a back surface. The liquid removal system of the present invention is characterized in that said interface device weighs less than 500 grams.

### Detailed description of the invention

Materials suitable for the membrane of the present invention and suction sources suitable for the liquid removal system of the present invention are described for example in United States patents No. 5,678,564 (Lawrence et al.) No. 5,911,222 (Lawrence et al.) incorporated herein by reference. Preferably, the membrane material can be bend to an extend which includes most of the typical in-use conditions without substantially loosing its functionality. More preferably, the membrane material of the present invention has a low bending moment in the longitudinal and/or the transverse direction in order to improve the comfort of the system. Preferably, the suction source as a whole or at least those parts connecting the suction source with the second zone of the liquid removal system are chosen to be flexible and/or compressible in order to improve the comfort of the system.

For the purpose of the present invention, a locally Cartesian coordinate system is defined relative to the liquid removal system and its positioning relative to the wearer during use. The longitudinal or x -- direction is defined as the direction running from the front waist region of the wearer to the back waist region of the web. Typically, the longitudinal direction is the longest dimension of the liquid removal system. The transverse or y -- direction is defined as direction running from the left side of the wearer to the right side of the wearer. The z -- direction is normal to the x -- direction and to the y -- direction and accordingly is also substantially normal to the body surface of the wearer during use. It is to be understood in this context that during wear of the liquid removal system of the present invention the liquid removal system conforms to the body shape of the wearer and that accordingly the coordinate axis at the front region of the liquid removal system may not coincide with the coordinate axis in the back region of the liquid removal system.

The interface device of the liquid removal system of the present invention weighs less than 500 grams, preferably less than 400 grams, more preferably less than 300 grams, even more preferably less than 200 grams, most preferably less than 100 grams. This weight is to be understood as being after activation, i.e. after the membrane has been wetted with the first liquid, and before use, i.e. before the second liquid enters the first zone. Preferably, the weight of the interface device is maintained during use when the second liquid and any further liquid enters the first zone and is absorbed into the liquid removal system. Since the urine is acquired through the interface device, this device has to be located close to the urethral exit of the wearer during use. The weight of the interface device of the present invention accordingly improves the wearing comfort when the interface device is positioned close to the urethral exit of the wearer.

One way to reduce the weight of the interface device of the liquid removal system is to minimize the amount of liquid present in the activated interface device. Preferably, the interface device of the present invention contains less than 300 milliliters of liquid in its activated state, preferably less than 200 milliliters of liquid in its activated state, more preferably less than 100 milliliters of liquid in its activated state, and most preferably less than 50 milliliters of liquid in its activated state. The amount of liquid in the interface device can be reduced by decreasing the liquid accessible volume inside the interface device. Alternatively, the weight of the interface device may be reduced by using light weight flexible support means and special or backsheet material for the construction of the second zone. Preferably, the flexible support the and the backsheet material comprised in the second zone have a combined weight of less than 200 grams, more preferably of less than 100 grams, most preferably of less than 50 grams. Suitable lightweight materials to manufacture the flexible support means and/or the liquid impermeable material are well known in the art.

The liquid removal system of the present invention preferably has a crotch dimension of less than 100 mm, more preferably of less than 75 mm, most preferably of less than 50 mm. In this context, the crotch dimension is the transverse dimension of a part of liquid removal system which is positioned adjacent the urethral exit of the wearer during use. Typically, this part will be at least a portion of the interface device of the present invention. Due to the anatomy of the wearer, only a limited space in the transverse direction is accessible for placement of the liquid removal system opposite the urethral exit of the wearer. Accordingly, a reduction in the crotch dimension will yield increased wearing comfort for the wearer.

The interface device of the liquid removal system of the present invention preferably has a longitudinal dimension less than 200 mm, more preferably of less than 150 mm, even more preferably of less than 100 meters, most preferably of less than 50 mm. In particular for mobile patients, a reduced longitudinal dimension of the interface device increases the wearing comfort and allows for concealed wearing of the liquid removal system of the present invention.

The liquid removal system of the present invention preferably has a thickness dimension of less than 30 mm, more preferably of less than 20 mm, most preferably of less than 10 mm. The thickness dimension in this context is considered to be the z -- dimension of the part of the liquid removal system which is positioned in proximity to the crotch region of the wearer. Typically, this part will be at least a portion of the interface device of the present invention. In particular for mobile patients, a reduced thickness dimension of the liquid removal system of the present invention increases the wearing comfort and allows for concealed wearing of the liquid removal system of the present invention.

The interface device of the liquid removal system of the present invention preferably has a user facing surface having a surface area of less than 50 square centimeters, more preferably less than 40 square centimeters, and most preferably less than 30 square centimeters. In particular from about patients, a reduced surface area of the user facing surface increases the wearing comfort and allows for concealed wearing of the liquid removal system of the present invention.

As is readily apparent to the skilled person, the reduction of weight and the reduction of any dimension can only be achieved by providing a liquid removal system exhibiting a sufficient performance for example in terms of acquisition rate, liquid transportation rate, storage capacity, membrane performance, and the like.

In the following, a suitable embodiment of the liquid removal system and of suitable members for a liquid removal system respectively will be described. The liquid removal system is assembled from an open celled foam material which is completely enveloped by a membrane. A suitable membrane material is available from SEFAR of Rüschlikon, Switzerland, under the designation SEFAR 03-20/14. A suitable foam material is available from Recticel of Brussels, Belgium, under the designation Bulpren S10 black. A suitable technique to completely envelope the foam material with the membrane material is to wrap the membrane material around the foam material and to subsequently heat seal all open edges of the membrane material. It will be readily apparent to the skilled practitioner to choose other similarly suitable materials. Depending on the specific intended application of the liquid removal system, it may also be required to choose similar materials with slightly different properties. After assembly, the liquid removal system is activated by immersing the liquid removal system in water or in synthetic urine until the liquid removal system is completely filled with liquid and until the membranes are completely wetted with liquid. After activation, a part of the liquid inside the liquid removal system may be squeezed out by applying an external pressure to the liquid removal system. If the activation of the liquid removal system was successful, the liquid removal system should not suck air through the membranes.

The particular geometry of the liquid removal system of the present invention can be varied to according to the specific requirements off the intended application. If, for example, the liquid removal system is intended to be used in an absorbent article the liquid removal system may be defined such that its zone of intended liquid acquisition fits between the legs of the wearer and further that its intended liquid discharge zone matches the form of the storage member associated to it. Accordingly, the outer dimensions of the liquid removal system such as length, width, or thickness may also be adapted to the specific needs of the intended application. In this context, it has to be understood , however, that the design of the outer form of the liquid removal system may have an impact on its performance. For example, the cross section of the liquid removal system directly impacts on its flow rate.

For application of the liquid removal system in an absorbent article according to the present invention, the liquid removal system is combined with a storage member. The term "liquid storage member" refers to a device which is capable of acquiring and storing liquid. The volume of the liquid storage member may vary with the amount of stored liquid such as by swelling. Typically, the storage member will imbibe the liquid by means of capillary suction and/or osmotic pressure. Other storage members may also use vacuum as a means to store the liquid. The liquid storage member is further capable of holding at least a portion of the stored liquid under pressure. Suitable storage members are well known in the art and may comprise for example a super absorbent polymeric material such as polyacrylate. The storage member may further comprise a fibrous structure, such as a pad of cellulosic fibers, in which the particulate superabsorbent material is dispersed. In order to pick up the liquid discharged from the liquid removal system, the storage member may be placed in direct liquid communication with the intended liquid discharge zone of the liquid removal system. A suitable storage member is for example a superabsorbent polymer such as available from CHEMDAL, United Kingdom, under the designation ASAP400.

Further examples of suitable superabsorbent polymers, often also referred to as "hydrogel forming polymer" or "absorbent gelling material", are described in U.S. Patent 5,562,646 (Goldman et al.), issued Oct. 8, 1996 and U.S. Patent 5,599,335 (Goldman et al.), issued Feb. 4, 1997.

Other liquid removal systems suitable for the purposes of the present invention are described for example in the PCT patent application No. PCT/US98/13497 entitled "Liquid transport member for high flux rates between two port regions" filed in the name of Ehrnsperger et al. filed on June 29, 1998, and in the following PCT patent applications PCT/US99/14796 entitled "High flux liquid transport members comprising two different permeability regions" (P&G case CM1840MQ) filed in the name of Ehrnsperger et al., PCT/US99/14654 entitled "Liquid transport member for high flux rates between two port regions" (P&G case CM1841MQ) filed in the name of Ehrnsperger et al., PCT/US99/14638 entitled "Liquid transport member for high flux rates against gravity" (P&G case CM1842MQ) filed in the name of Ehrnsperger et al., PCT/US99/14633 entitled "Liquid transport member having high permeability bulk regions and high bubble point pressure port regions" (P&G case CM1843MQ) filed in the name of Ehrnsperger et al. All of these documents are enclosed herein by reference.

In one embodiment of the present invention, the liquid removal system of the present invention is geometrically saturated or substantially geometrically saturated with free liquid. The term "free liquid" as used herein refers to liquid which is not bound to a specific surface or other entity. Free liquid can be distinguished from bound liquid by measuring the proton spin relaxation time T₂ of the liquid molecules a according to NMR (nuclear magnetic resonance) spectroscopy methods well known in the art.

The term "geometrically saturated" as used herein refers to a region of a porous material in which the liquid accessible void spaces have been filled with a liquid. The void spaces referred to in this definition are those which are present in the current geometric configuration of the porous material. In other words, a geometrically saturated device may still be able to accept additional liquid by and only by changing its geometric configuration for example by swelling, although all voids of the device are filled with liquid in the current geometric configuration. A device for handling liquids is called geometrically saturated, if all porous materials that are part of the device and intended for liquid handling are geometrically saturated.

The term "porous material" as used herein refers to materials that comprise at least two phases a solid material and a gas or void phase - and optionally a third liquid phase that may be partially or completely filling said void spaces. The porosity of a material is defined as the ratio between the void volume and the total volume of the material, measured when the material is not filled with liquid. Non-limiting examples for porous materials are foams such as polyurethane, HIPE (see for example PCT patent application WO94/13704), superabsorbent foams and the like, fiber assemblies such as meltblown, spunbond, carded, cellulose webs, fiber beds and the like, porous particles such as clay, zeolites, and the like, geometrically structured materials such as tubes, balloons, channel structures etc. Porous materials might absorb liquids even if they are not hydrophilic. The porosity of the materials is therefore not linked to their affinity for the liquid that might be absorbed.

The term "substantially geometrically saturated" as used herein refers to a member in which at least 90% of the macroscopic void volume of the member are geometrically saturated, preferably at least 95% of the macroscopic void volume of the device are geometrically saturated, more preferably 97% of the macroscopic void volume of the device are geometrically saturated, most preferably 99% of the macroscopic void volume of the device are geometrically saturated.

In one embodiment of the present invention, the absorbent article is a disposable absorbent article such as a diaper, a training pant, a sanitary napkin, an adult incontinence article, or the like. Such an absorbent article may further comprise a liquid pervious topsheet, a liquid impervious backsheet at least partially peripherally joined to the topsheet. The absorbent article may further comprise an absorbent core which may serve as a storage member for the urine. Topsheets, backsheet, and absorbent cores suitable for the present invention are well known in the art. In addition, there are numerous additional features known in the art which can be used in combination with the absorbent article of the present invention such as for example closure mechanisms to attach the absorbent article around the lower torso of the wearer.

## Claims

1. A liquid removal system which comprises
-- an interface device, said interface device comprising
-- a first zone and
-- a second zone and having
-- a porous membrane separating said first zone from said second zone,
said second zone being adapted to be connected to a suction source,
said porous membrane being capable of maintaining a suction in said second zone without permitting air
from said first zone to pass through said membrane into said second zone when said membrane has been
wetted with a first liquid; said suction being maintained until said membrane is contacted with a second
liquid;
and wherein said second liquid upon entering said first zone and contacting said porous membrane is
removed from said first zone by said suction in said second zone by passing through said membrane into
said second zone
said first zone having a user facing surface, said second zone having a back surface,
characterized in that
said interface device weighs less than 500 grams.

2. A liquid removal system according to claim 1
wherein
said interface device contains less than 300 milliliters of liquid in its activated state.

3. A liquid removal system according to claim 1 or to claim 2,
said second zone comprising a flexible support means and being covered by a flexible, liquid impermeable material
wherein
said flexible support means and said liquid impermeable material have a combined weight of less than 200 grams.

4. A liquid removal system according to any of the preceding claims,
said interface device having a crotch dimension
wherein
said crotch dimension is less than 100 mm.

5. A liquid removal system according to any of the preceding claims
said interface device having a longitudinal dimension
wherein
said longitudinal dimension is less than 200 mm.

6. A liquid removal system according to any of the preceding claims
said interface device having a thickness dimension
wherein
said thickness dimension is less than 30 mm.

7. A liquid removal system according to any of the preceding claims
wherein
said user facing surface has a surface area of less than 50 square centimeters.
